# EUROPEAN PATENT SPECIFICATION

(11) **EP 1 506 759 B1**
(45) Date of publication and mention of the grant of the patent: **12.08.2009**
(21) Application number: 04027741.0
(22) Date of filing: 03.06.1996
(51) Int. Cl.: A61F 11/06, A61F 11/14

(54) **A covering device and an ear warmer**
Ohrenschützer und -wärmer
Protecteur et réchauffe-oreilles

(30) Priority: 02.06.1995 US 460587
(43) Date of publication of application: 16.02.2005
(62) Divisional of application: 02013409.4
(73) Proprietor: 180s, Inc., Baltimore Maryland 21202-3101 (US)
(72) Inventor: Legette, Brian E., Severna Park Maryland 21224-3654 (US); Wilson II, Roland L., Parkersburg, WV 26104-1705 (US)
(74) Representative: Grünecker, Kinkeldey, Stockmair & Schwanhäusser Anwaltssozietät

(56) References cited:
- CH-A5- 662 052
- FR-A- 2 536 253
- US-A- 2 615 169
- US-A- 2 776 436
- US-A- 3 249 949
- US-A- 3 787 899
- US-A- 5 257 420

## Description

### Field of the Invention

The present invention relates to a covering to be worn over and to protect the ears of an individual. The covering is intended to extend around the back of the head or neck of the individual when being worn. The present invention also relates to an ear protection device that may incorporate audio earphones or similar type elements.

### Background of the Invention

There are a number of prior devices which cover an individual's ears for warmth or the like. These devices, generally known as "ear muffs". are represented in part by U.S. Pat. No. 3,249,949 to Rosenberg et al. and U.S. Pat. No. 3.509.580 to Rubenstein et al. These prior ear muffs generally include a band which wraps around the top of the head and includes an enlarged end which engages and covers the ears for warmth and protection.

An alternate method for securing ear protection type structures is shown in Rosenberg U.S. Pat. No. 2,070,216 which is in the form of a headband. Edwards U.S. Pat. No. 5,201,856 shows a device which wraps around the side of an individual's head and attaches at the front end to the arms of eyeglasses. Brinkley U.S. Pat. No. 5,339,467 shows an car protection type device which attaches to the rim of the ear and maintains its position without a securing strap or the like.

Ballard U.S. Pat. No. 548,738 shows an ear and neck protecting device which wraps around the back of the individual's head and covers the neck to a position below the collar. Triplett U.S. Pat. No. 2,671,221 shows an ear protection device that wraps around the back of an individual's head and includes a hinge at its center.

It has previously been contemplated that audio speakers may be incorporated into ear muff or headband type devices. Krawangna U.S. Pat. No. 3,787,899 shows an ear muff that includes audio speakers therein. Spates U.S. Par. No. 4,864,619 shows a stereo head set within a headband. A wrap for a standard headphone is shown in Byme, Jr. U.S. Pat. No. 5,257,420.

US 2,776,436 relates to ear muffs and discloses all of the features of the preamble of claim 1.

US 3,249,949 relate to the earmuffs comprising an arcuate head band including two free end portions to which two earpieces are mounted

Despite the existence of various types of ear muffs and headbands, there remains a need for an ear protection device having the advantages of the present invention.

### Summary of the invention

The present invention provides a covering device to be worn over the ears of an individual according to claim 1. Preferably, the covering device extends between the opposite ears around the back of the individual's head or neck.

One embodiment of the covering device of the present invention includes a flexible band which is positioned between two ear cups. The band includes a central curved portion and two end portions. The car cups are attached to the end portions of the band and project therefrom. At least one portion of the ear cups includes a frusto conical frame member having a central opening therein. A fabric is used to cover at least the frusto conical portion and central opening of the ear cups so as to from a covering for the ears.

The fabric which covers the ear covering device may cover both the ear cups and the band. The fabric may include an opening within the portion covering die ear cups so that a earphone or the like may be inserted into the pocket or cavity formed by the frusto conical frame portion and the fabric covering. The ear cups may also be hingedly connected to the ends of the band and the band may be collapsible.

Another embodiment of the covering device of the present invention includes a band having two curved portions with one end of each curved band portion overlapping and slideably attached to the other curved band portion such that the relative overall length of the band may be adjusted. Two ear cup portions are attached to the free ends of the curved portions, the ear cap portions having a frusto-conical part, wherein the band and ear cup portions are covered by a fabric means. The ear cups may include V-shaped support flanges. A semi-circular frame extends between the projections of the V-shaped portion. A curved ear cup extension portion extends from each of the projections of the V-shape in a direction opposite of the semi-circular portion. These portions of the ear cup form an ear cavity. The semi-circular portion and the support flanges also define a central opening in the ear cavity. An attachment flange projects from the vertex of the V-shaped support flanges. The attachment flange including means thereon for rotatably attaching the ear cups to the free ends of the curved band portions. Fabric means covers the band and the ear cups on both sides and forms a pocket or cavity adjacent the central opening in the ear cups.

The invention further contemplates the use of specific dimensional relationships between the ear cups and the band, as well as specific materials for the construction of the covering device, so as to form a comfortable and functional ear covering device which may be worn by almost any individual for protection of the ears.

According to a further embodiment, a covering device to be worn over the ears of an individual and extend around the back of the individual's head comprises:
a curved flexible band, the band including a first curved portion and a second curved portion, one end of each of the first and second curved portions overlapping and slidably attached to one another such that the relative overall length of the band may be adjusted;
two ear cup frame members, one ear cup frame connected to the free end o each curved portion of the band, opposite the overlapping attachment of the curved portion to the other curved portion, at least a portion of the ear cups having a central opening therein and defining a cavity for receipt of an individual's ear therein; and
fabric means covering at least the frusto conical portion of the ear cups and the central opening.

In a further embodiment, the fabric means covers the ear cups and the flexible band.

In a further variation of the above embodiments, the ear covering device further comprises opening means formed in the fabric means for insertion of an earphone or the like into the cavity.

In a further embodiment, a portion of the speaker wire is retained within the fabric means covering the band.

In a further embodiment of any of the preceding embodiments, the ear cup frame member comprises an attachment flange for attaching the ear cup to the portion.

In a further embodiment, the attachment flange of the ear cup frame member includes a hinge for permitting at least partial rotation of the frusto conical portion about the attachment flange.

In a further embodiment, the hinge is a living hinge.

In a further embodiment, the hinge connection includes means for limiting the rotation of the ear cup frame member about the attachment flange.

In a further embodiment, the attachment of the ear cups to the opposite end of the band is formed by the rotational pivot, such that the ear cups may rotate about an axis of the attachment and the opposite ear cups may be rotated to be substantially parallel to one another.

In a further embodiment, the rotational pivot attachment of the ear cups to the end of the band is formed by an attachment head, the attachment head pivot permitting the ear cups to rotate about the axis of the attachment head.

In a further embodiment, the ear cup portions include two support flanges formed in a V-shape, and a semi-circular frame portion which extends between opposite projections of the V-shape of the flanges, the semi-circular portion and the support flanges defining the central opening.

In a further embodiment, the ear cup portions further comprise an attachment flange projecting from the vertex of the support flanges in a direction opposite the semi-circular portion, the attachment flange including means thereon for rotatably attaching the ear cups to the free ends of the first and second curved portions.

In a further embodiment, the ear cup portions further comprises two cup extensions projecting from the projected ends of the V-shaped support flanges in a direction opposite of the semi-circular frame portion.

In a further embodiment, the attachment means for the ear cups further comprises an attachment head which projects from the outer surfaces of the attachment flange, the attachment head forming a pivot for the ear cups with respect to the free end of the band portions upon which it is attached, the attachment permitting rotation of the ear cups about the axis of the attachment head.

In a further embodiment, the attachment head is snap-fit within an opening of the end of the band portion.

In a further embodiment, the first curved portion of the band includes a series of raised bumps on the interior surface thereof which engage the overlap formed by the second band portion to form a resistance to the relative sliding movement of the two band portions when adjustment of the overall length of the band is desired.

In a further embodiment, an ear warmer frame, comprises a band having a first curved portion having an enlarged portion at a first end and a passageway at a second end, and a second curved portion having an enlarged portion at a first end and a passageway at a second end, each passageway having a first slot portion allowing the opposite curved portion of said band to slide through and having a second slot portion allowing the enlarged portion of the opposite curved portion of said band to slide through, whereby one end of each of the first and second curved portions overlapping and slidably attached to opposing curved portion, such that the relative overall length of the band may be adjusted.

A further embodiment further comprises two ear cups each having an attachment head, each attachment head attaching to one enlarged portion of one curved portion of said band.

A further embodiment the passageway of the first curved portion of said band is integrally formed with the first curved portion of said band, the passageway of the second curved portion of said band is integrally formed with the second curved portion of said band.

In a further embodiment, the first curved portion of the band includes a series of raised bumps on the interior surface thereof which engage the overlap formed by the second band portion to form a resistance to the relative sliding movement of the two band portions when adjustment of the overall length of the band is desired.

In a further embodiment, a covering device to be worn over the ears of an individual and extending around the back of the individual's head or neck, comprises a band having a central curved portion and two opposite end portions, two ear cups , each ear cup attached to the opposite end portions of the band, the attachment being formed by an attachment flange, each ear cup having a cup portion having a central opening therein, the curvature of the central portion of the band and the attachment flange with the cup portion directing the ear cups inwardly towards one another from the opposite ends of the band, and fabric means covering the band and the ear cups on both sides.

In a further embodiment, the attachment flange is integrally formed with the cup portion at an oblique angle thereto.

In a further embodiment, the fabric means forms a pocket within the cup portion of the ear cups.

In a further embodiment, said band has a first curved portion and a second curved portion, one end of each of the first and second curved portions overlapping and slidably attached to one another such that the relative overall length of the band may be adjusted, said two ear cups are attached to the ends of the first curved portion and the second curved portion opposite their overlap, respectively, the attachment of the ear cup to the band permits rotation of the cup portion about the attachment.

In a further embodiment, the fabric means envelopes the band and the ear cups.

The cup portion has a frusto conical shape.

In a further embodiment, said band has a first curved portion and a second curved portion, one end of each of the first and second curved portions overlapping and slideably attached to opposing curved portion, such that the relative overall length of the band may be adjusted, said two ear cups are attached to the free end of the first curved portion and to the free end of the second curved portion, respectively, the ear cups defining a cavity formed from two support flanges, and a semi-circular frame portion which extends between opposite projections of the support flanges, the semi-circular portion and the support flanges defining a central opening, the attachment flange projecting from the support flanges in a direction opposite the semi-circular portion, the attachment flange including means thereon for rotatably attaching the ear cups to the free ends of the first and second curved portions.

In a further embodiment, the two support flanges are formed in a V-shape, and the attachment flange projects from the vertex of the support flanges.

In a further embodiment, the fabric means forms a pocket within the cavity of the ear cups.

In a further embodiment, the fabric means is constructed from a plurality of fabric pieces.

In a further embodiment, the two ear cups rotatably engage into a position at the free ends of the first and second curved portions so that the ear cups are aligned with the band.

In a further embodiment, the band and the two support flanges direct the ear cups inwardly towards one another to maintain position of the covering device.

In a further embodiment, the covering device retains the position around the back of the individual's head or neck without any support around the top of the individual's head.

In a further embodiment, an ear warmer frame, comprises a band having a first curved portion having an enlarged portion at a first end and a passageway at a second end, and a second curved portion, having an enlarged portion at a first end and a passageway at a second end, each passageway having a first slot portion allowing the opposite curved portion of the said band to slide through and having a second slot portion allowing the enlarged portion of the opposite curved portion of said band to slide through, whereby one end of each of the first and second curved portions overlapping and slidably attached to opposing curved portion, such that the relative overall length of the band may be adjusted.

In a further embodiment, the ear warmer further comprises two ear cups each having an attachment, head, each attachment head attaching to one enlarged portion of one curved portion of said band.

In a further embodiment, the passageway of the first curved portion said band is integrally formed with the first curved portion of said band, the passageway of the second curved portion said band is integrally formed with the second curved portion of said band.

In a further embodiment, the first curved portion of the band includes a series of raised bumps on the interior surface thereof which engage the overlap formed by the second band portion to form a resistance to the relative sliding movement of the two band portions when adjustment of the overall length of the band is desired.

In a further embodiment, a covering device to be worn over the ears of an individual and extend around the back of the individual's head comprises a flexible band, the band having a central curved portion and two end portions, two ear cups, one ear cup attached to each end portion of the band, at least a portion of the ear cups having a frusto conical configuration and a central opening therein, and fabric means covering the ear cups and the flexible band.

In a further embodiment, the fabric means and the ear cups form a pocket, whereby opening means are formed in the fabric means for insertion of an earphone into the pocket.

In a further embodiment, the flexible band has an inner side and an outer side, each ear cup has an inner side and an outer side, and the fabric means covers both the inner side and the outer side of the flexible band and covers both the inner side and the outer side of each ear cup.

In a further embodiment, the ear cups are hingedly connected to the end portions of the band.

In a further embodiment, the hinge connection between the end portions and the band is formed by a living hinge.

### Brief Description of the Drawings

For the purpose of illustrating the invention, there is shown in the drawings a form which is presently preferred; it being understood, however, that this invention is not limited to the precise arrangements and instrumentalities shown.
Figure 1 shows one form of the covering device contemplated by the present invention as being worn by an individual.
Figure 2 shows a top elevational view of an ear covering device contemplated by the present invention.
Figure 3 shows a side plan view of the frame of the covering device of the present invention.
Figure 4 shows a front plan view of the frame of the covering device of the present invention.
Figure 5 shows a bottom elevational view of the frame of the covering device of the present invention.
Figure 6 shows the covering device incorporating a headphone type speaker arrangement therein.
Figures 7A and 7B show the component parts of the covering device and headphone speakers as contemplated by Figure 6.
Figures 8A through 8F show a series of covering panels which form the fabric covering for the present invention.
Figures 9 through 15 show various embodiments of a hinged connection for use with the ear covering device of the present invention.
Figures 16 through 24 show side elevational views of various embodiments of a "living hinge" for use within the covering device of present invention.
Figure 25 shows a collapsible frame for an ear covering device.
Figure 26 shows the collapsible frame of Figure 25 illustrated in an opened position.
Figure 27 shows an ear covering device in accordance with the present invention, illustrated in the collapsed position.
Figure 28 is a top plane view of a portion of an alternate embodiment of an ear covering device as contemplated by the present invention.
Figure 29 is a side elevation of the alternate embodiment of the ear covering device as shown in Figure 28.
Figure 30 shows a side elevation of the alternate frame embodiment as contemplated by Figures 28 and 29.

### Detailed Description of the Invention

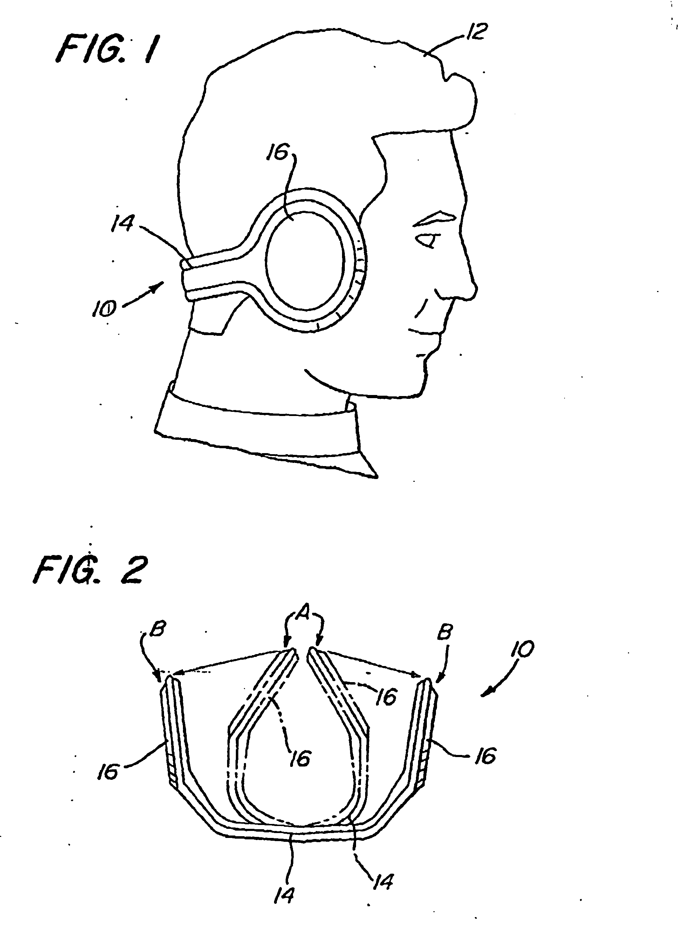

In the Figures, where like numerals indicate like elements, there is shown multiple forms of an ear covering device as contemplated by the present invention. The ear covering device as illustrated in Figure 1 is generally designated by the numeral 10. The ear covering device 10 is adapted to be worn over the ears of an individual 12. When worn, the device 10 extends around the back of the head and/or neck of the wearer.

The ear covering device 10 generally includes a band portion 14 and two ear protecting portions 16. One side of the ear covering device 10 is illustrated in Figure 1. The opposite side of device 10 is contemplated to be a mirror image of that illustrated in Figure 1. As illustrated in Figure 1, the band portion 14 is relatively narrower than the ear covering portions 16. It is contemplated that variations on this dimensional relationship may be made without departing from the essence of the present invention.

Figure 2 shows a top plan view of the ear covering device 10 illustrated in Figure 1. In position "A" in Figure 2, the ear covering device 10 is illustrated in its normal rest position with the ear covering portions 16 positioned closely adjacent to one another. The rest positioning of the covering portions is created by the curvature of band portion 14 and the form of the frame for the ear covering portions 16 (shown in detail in other figures). Position "B", as illustrated in Figure 2, shows the ear covering portions 16 deflected outwardly due primarily to a flexing of band 14. The enlargement of the distance between opposite ear covering portions 16 permits the car covering device 10 to be placed on the head of the individual 12, as illustrated in Figure 1. The spring force created by the flexible band 14 of the ear covering device 10 causes the ear covering portions 16 to engage against the side of the head of the individual 12. The spring force of the band is contemplated to be sufficient to engage the head 12 so that the ear covering device 10 does not fall off. However, the spring force is desired to be limited so that the ear covering device is not uncomfortable to wear.

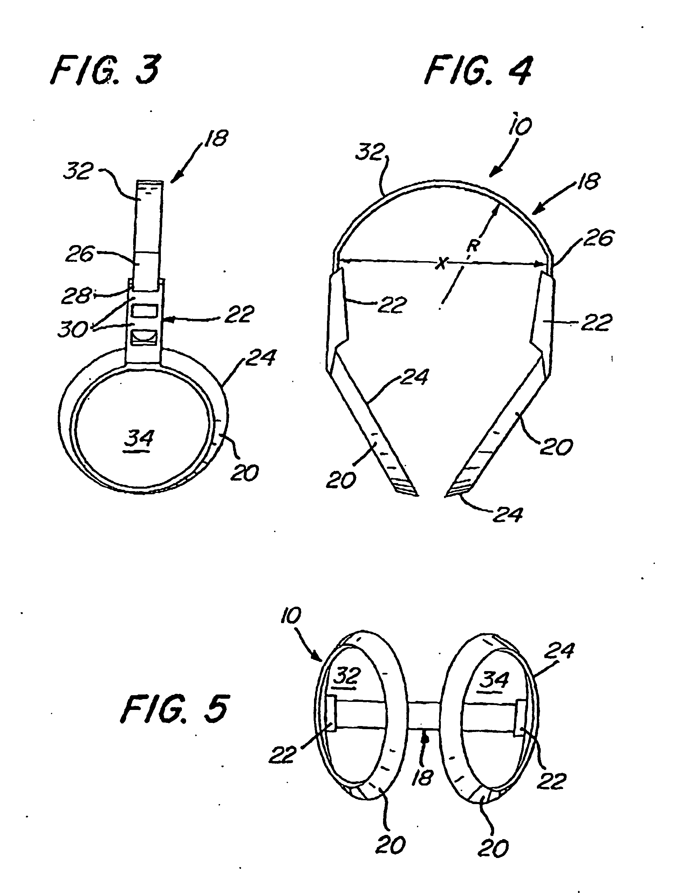

Figures 3-5 generally illustrate the framework of covering device 10. The frame generally comprises a flexible band 18 and two ear cup frame members 20. The ear cup frame members 20 include an attachment portion or flange 22 and a frusto conical frame portion 24. The attachment portion 22 in the embodiment illustrated in Figures 3-5 is integrally formed with the frusto conical portion 24. The attachment portion 22 is formed at an oblique angle with respect to the plane of the frusto conical portion 24. This angle creates, in part, the inward positioning of the car covering portions 16 as illustrated in Figure 2, i.e., position "A". The angle between the attachment flange 22 and the frusto conical portion 24 is contemplated to be in the range of about 90 to 160° and is preferably approximately 145°. The flexible band 18 includes end portions 26 which are engaged within the attachment portions 22 of the ear cup frame members 20. As illustrated in Figure 3, the end portion 26 is engaged within a slot 28 within attachment portion 22 and is secured therein by ribs 30. A detent (not shown) may be provided on the end portion 26 of the flexible band 18 so as to resist the removal of the ear cup frame member 20 from between the opposing ribs 30 on the end portion 26.

The flexible band 18 as illustrated includes a curved portion 32 and two end portions 26. Preferably, the curved portion 32 has a radius of curvature of approximately 2 1/4 inches. The end portions are formed by bends at the end of the curved portion 32 and are separated by a distance X, as illustrated in Figure 4. Preferably, distance X is in the range of about 3.5 to 4.5 inches and specifically about 3.9 inches. The end portions 26 are straight and are formed integral with the curved portion 32. Preferably, the band 18 is made of a stainless steel type material; however, other materials are contemplated, including polypropylene, nylon, polyethylene, etc. The band 18 should be sufficiently flexible such that the relative distance X between the two end portions 26 may be enlarged and the ear cup frame members 20 may be separated prior to placing ear covering device 10 on the individual 12 (Figure 1).

The ear cup frame members 20 are preferably made of a plastic material, such as nylon. The frusto conical portions 24 at each end of the frame include a central opening 34. The diameter of the frusto conical portion at its base is contemplated to be in the range of about 2.7 to 4 inches and preferably in the neighborhood of 3 inches. The preferred height of the frusto conical portion is in the range of about 0.2 to 0.5 inches. The preferred height dimension if approximately 0.25 inches with the side wall being at an angle of about 45°. The frusto conical portion 24 is contemplated to fit around the ear when wearing of the ear covering device 10.

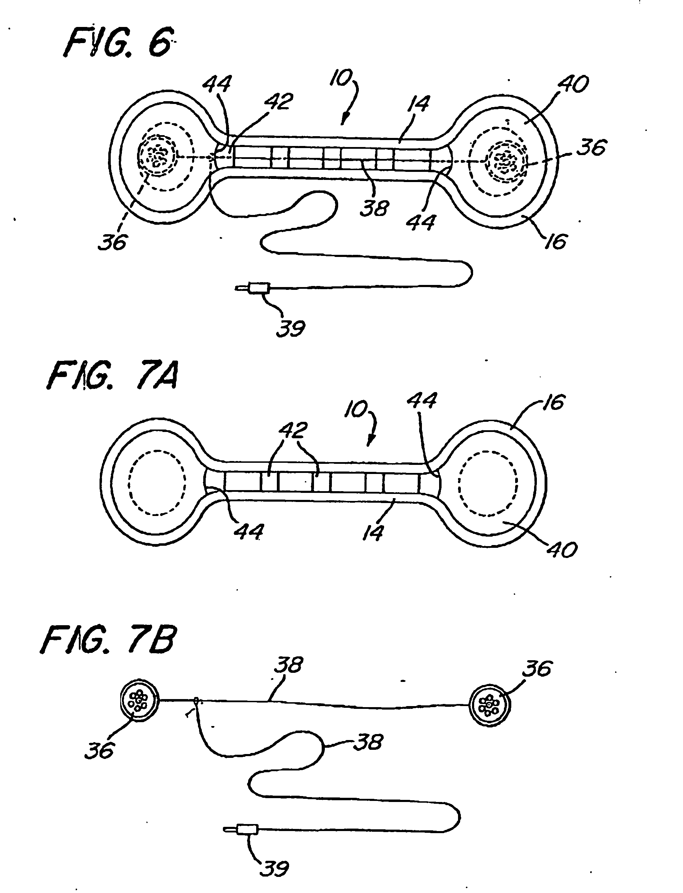

In Figure 6, the ear covering device 10 is shown having a pair of speakers or earphones 36 and a speaker wire 38 incorporated therein. The earphones 36 and speaker wire 38 are also illustrated in Figure 7B, separate from the ear covering device 10. The speaker wire 38 terminates in a standard male jack 39 for connection to a personal radio, tape player or the like (not shown).

In Figures 6 and 7A, the ear covering device 10 includes band 18 and ear cup frame members 20 as shown in Figures 3-5. The band 18 and ear cups 20 are covered by a fabric material 40. As illustrated, the band portion 14 and the ear covering portions 16 are each covered with fabric 40. The fabric covering 40 includes a series of bands 42 which engage the speaker wire 38 and retain it within the ear covering device 10. In addition, a pocket on cavity is formed within the car cups 20 which retains the earphones 36. The earphones 36 may be inserted into the pocket or cavity by means of slot 44 within the fabric 40. As illustrated, the slot 40 is positioned adjacent to each ear covering portion 16.

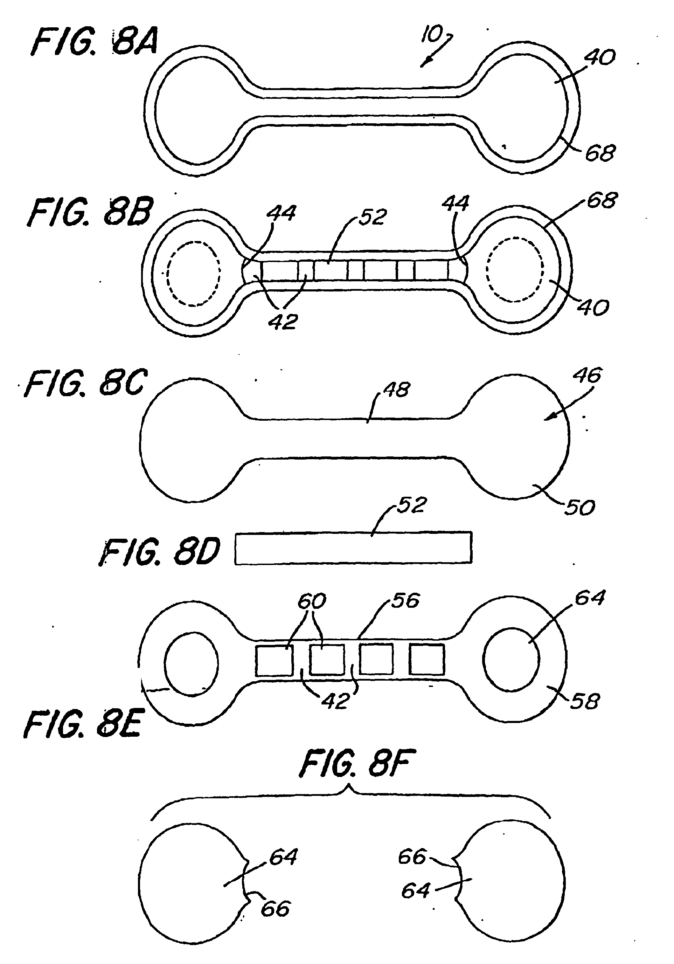

As generally illustrated in Figures 8A-8F, the fabric 40 includes multiple layers. In Figure 8A, there is shown a top plan view of the ear covering device 10 in the open position. The fabric 40 completely covers the outside surfaces of the band 18 and the car cup frame members 20. As shown in Figure 8B, the fabric 40 also covers the inside surfaces of the band 18 and ear cups 20.

Multiple layers of fabric are utilized to form the fabric covering 40 for the car covering device 10. These layers are generally illustrated in Figures 8C-8F. In Figure 8C, there is shown an outer shell 46 which includes a central tapered portion 48. In Figure 8D there is shown an inner fabric covering 52 which when assembled is exposed between bands 42, as shown in Figure 6. The inner fabric 52 generally covers the inner surfaces of band 18. The inner fabric 52 is overlapped by a central portion 56 which is shown in Figure 8E. The central portion 56 includes a series of alternating bands 42 and openings 60 in its center and two circular portions 58 at each end. A central opening 62 is provided within the circular portions 58. The central portion 56 is contemplated to be provided on the inside of the ear covering device 10 and at least partially covering the inner fabric 52. Covering the circular portions 58 of the band fabric 56 are inner ear members 64. The inner ear members 64 as shown in Figure 8F have a generally circular configuration. The inner ear members 64 are positioned over top of the circular portions 58 and the corresponding central opening 62 in central fabric 56. Adjacent one end of the inner ear members 64 is a curved border 66 which in the final constructions forms the slot 44 for insertion of the earphones 36 as contemplated by Figures 6 and 7.

The various layers of the fabric material 40 are combined by sewing or the like. The fabric covering may include an outer piping 68. Piping 68 may be formed as part of one of the layers of the fabric material 40 or may be a separate element attached thereto.

Any number of materials are contemplated for the fabric 40. Piping 68 as illustrated in Figures 8A and 8B may be a wear-resistant cotton blend or an entirely synthetic material. The exposed outer shell 46 may be a nylon-covered close cell neoprece or other moisture-resistant fabric. The band fabric 54 may also be made of neoprene. Inner central tapered fabric 52 and inner ear members 64 are preferably made of a synthetic fleece type lining material, so as to provide warmth and comfort. The inner ear members 64 will be in direct contact with the individual 12. It is noted that the exposed outer shell 46 may alternatively be made of a fleece material. It has been determined that the fleece material provides sufficient protection from the elements while also permitting sound to pass therethrough.

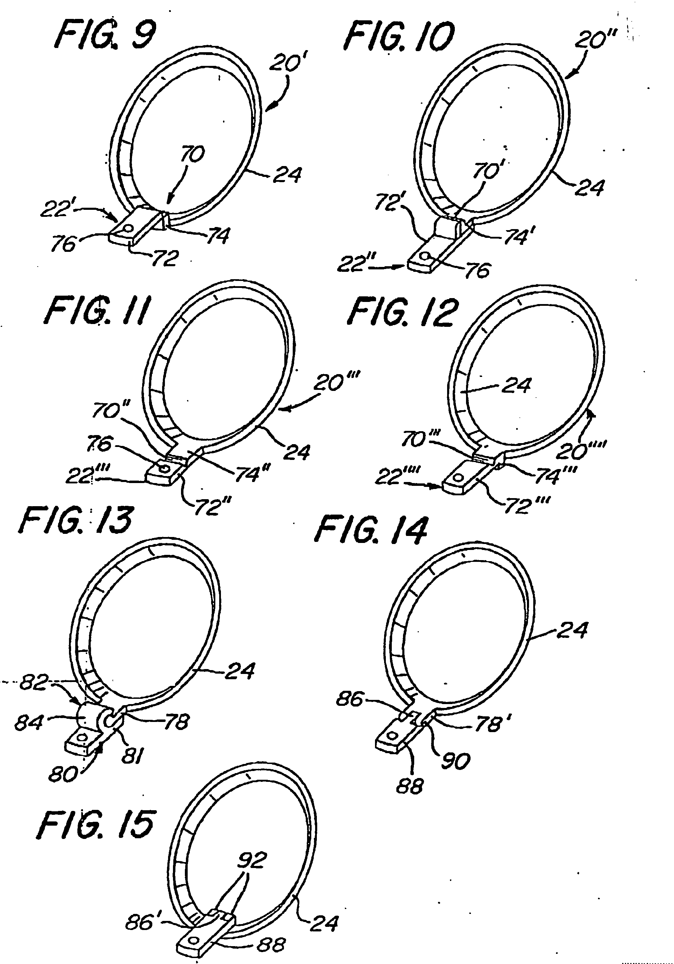

In Figures 9-23, there is illustrated a number of hinge embodiments which may be used to form the connection between the attachment portion 22 and the frusto conical portion 24 of the ear cup frame members 20. Each of these embodiments will be discussed separately below. A number of these embodiments include a living hinge type arrangement. A living hinge as contemplated by the present invention generally includes a continuous plastic formation having a reduced cross-section between a base flange and an attachment flange, wherein flexing is permitted between the two flanges. The form of each of the hinges will be discussed in further detail below.

In Figure 9, there is illustrated an embodiment of the attachment portion 22' for the car cup frame member 20'. The frusto conical portion 24 as illustrated in this Figure 9 is generally contemplated to be the same as that illustrated previously. However, the communication between the frusto conical portion 24 and the attachment portion is not contemplated to be rigid. The living hinge 70 is provided between an attachment flange 72 and a base flange 74. The living hinge 70 permits the ear cup 20 to pivot such that the ear covering portion 16 may collapse into the band portion 14 to reduce the size of the ear covering device 10 when not in use. A rivet hole 76 is provided in the attachment flange 72. The engagement between engagement portion 22' and the flexible band 18 (not shown in Figure 9) may be made by means of a rivet (not shown). It is contemplated that rotation of the ear cup frame member 20' about the rivet or opening 76 further permits collapsing the car covering portions 16 into band 14.

In Figure 10 there is illustrated a further embodiment of the ear cup frame member 20" which includes a frusto conical portion 24 and a modified living hinge 70'. The attachment portion 22" generally includes an inverted T-shaped attachment flange 72' and an L-shaped base flange 74'. The connection between the T-shaped flange 72' and the L-shaped flange 74' forms the living hinge 70' and limits the amount of outward rotation of the frusto conical portion 24 with respect to the attachment portion 22". The hinge 70' permits the inward rotation of the frusto conical portion 24 towards the attachment portion 22" when collapsing the ear covering device 10. Again, a rivet hole 76 is provided in the attachment flange 72' for securing the attachment portion 22" to the end portion of the flexible band 18 (above).

In Figure 11, there is shown a further embodiment of an ear cup frame member 20'''. In this embodiment, the attachment flange 72" is generally planar with the base flange 74" and includes the hinge 70" positioned therebetween. A slot or groove is provided as part of the living hinge 70" such that a limited amount of inward rotation of the frusto conical portion 24 is permitted with respect to the attachment flange 72". Again. the attachment flange 72" may be secured to band 18 (above) by means of rivet hole 76 and a rivet (not shown).

In Figure 12 there is illustrated a still further embodiment of an ear cup frame member 20"" including a hinge 70''' formed as part of the attachment portion 22''''. The base flange 74''' in this embodiment generally includes an L-shaped member with one leg of the L-shape being generally planar with the attachment flange 72'''. The living hinge 70''' is provided between the L-shaped flange 74''' and the attachment flange 72''' with the projection of L-shaped flange 74"' serving to prevent full rotation of the frusto conical portion 24 with respect to the attachment flange 72'''.

In Figures 13-15, there are illustrated further hinge embodiments for the ear cup frame members. These hinged arrangements are generally contemplated to include a press fit relationship and/or a pin secured within adjacent elements.

In Figure 13, the frusto conical portion 24 includes a projecting tab 78. Tab 78 includes a founded end and forms the axis for the hinge 82. Tab 78 is formed integral with the frusto conical portion 24. The securing portion 80 of hinge 82 includes a planar base 81 and a curved projection 84. The curved projection 84 generally encapsulates the end of the projecting tab 78 such as a hinge is formed. The planar base 81 of the securing portion 80 generally limits outward rotation of the frusto conical portion 24. The curve portion 84 may also limit inward rotation about the hinge 82.

In Figure 14, the frusto conical portion generally includes a protecting tab 78' which includes a central slot. A tab 86 on the securing portion 88 is engaged within the slot in tab 78. A pin 90 forms an axis for the hinge and extends through the ends of tabs 78 and 86.

In Figure 15 there is shown a further embodiment of a hinge construction for the ear cup frame. The frusto conical portion 24 includes two projections 92 which are formed on the inside surface of the cone 24. The base members 92 engage on opposite sides of a projecting tab 86' which is formed as part of the securing portion 88. A pin (not shown) forms the axis for the hinge and extends through the projections 92 and the tab 86' on securing portion 88.

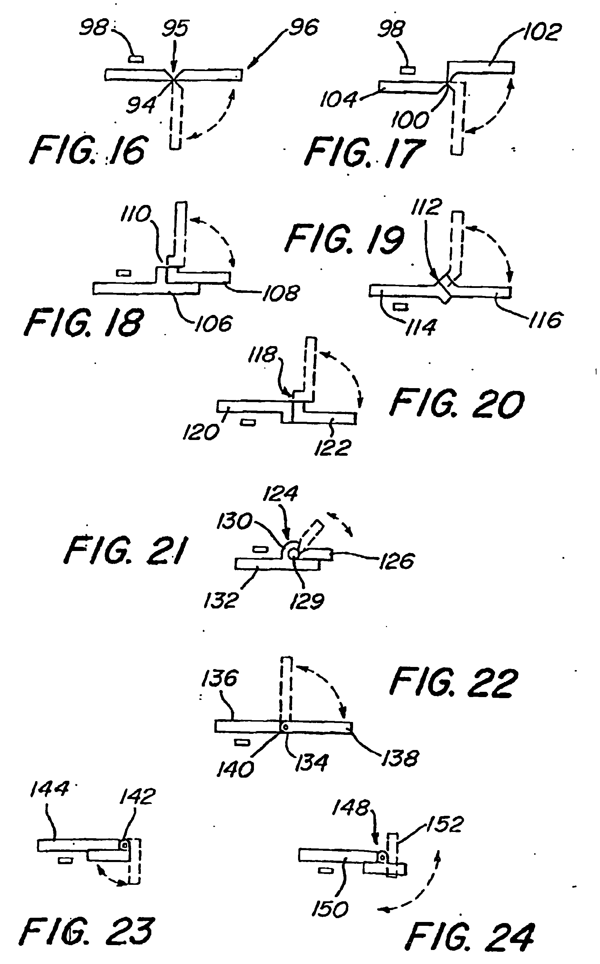

In Figure 16 there is illustrated a living hinge in the form similar to that shown in Figure 11. The hinge 94 generally includes a V-shaped notch 95 within a planar body portion 96. Projecting from the left hand element of the planar body portion 96 is an attachment head 98 which may be inserted into an opening in the end portion 26 of band 18 (not shown). It is contemplated that the press fit relationship between the opening in end portion 26 and the attachment head 98 will form a pivot similar to the rivet attachment contemplated for the embodiments in Figures 9-15. In phantom, there is illustrated the flexing of the living hinge 94.

In Figure 17, a further embodiment of the living hinge is shown. The hinge 100 is formed between a first base member 102 and a second base member 104. The second base member 104 includes an attachment head 98, similar to that contemplated by Figure 16. However, in this embodiment, the first base member is not planar with the second base member 104. The flexing of the hinge 100 is shown in phantom.

In Figure 18, there is illustrated a living hinge having a form similar to that shown in Figure 10 above. A first base member 106 forms an inverted T-shaped element and is provided along with an L-shaped base member 108. The hinge 110 is formed between the first and second base numbers 106, 108. Because of the projecting portion on the right hand side of the first base member 106 (as illustrated in Figure 18) the downward rotation of the second base member 108 is limited. The movement of the second base member 108 is shown in phantom.

In Figure 19, there is a shown a still further embodiment of a living hinge type attachment for the ear cup member. The hinge 112 is formed between a first base member 114 and a second base member 116. The first and second base members 114, 116 each include a bend at their respective ends which permit the formation of the hinge 112. The bent ends engage one another so as to limit rotation of second base member 116 in the downward direction (as shown). The rotation of member 116 in the upward direction is shown in phantom.

In Figure 20, there is a further embodiment of a living hinge type structure. In this embodiment, the hinge 118 is formed between two L-shaped members 120 and 122. The L-shaped members are positioned opposite one another, such that their elongated projections are not planar. This arrangement again limits downward rotation of the right hand L-shaped member 122. The upward rotation of member 122 is shown in phantom in this Figure 20.

In Figure 21, there is illustrated a hinge arrangement similar to than contemplated by Figure 13 above. The hinge 124 is formed by a tab 126 having a circular end 128 which is engaged within a curved projection 130 formed on a planar member 132. The tab 126 engages the upward surface of planar 132 so as to limit rotation thereof downwardly about the hinge 124. In addition, the upward rotation of tab 126 about the hinge 124 is limited as illustrated in phantom. The upper surface of tab 126 engages the end the of curved portion 130. The hinge 124 is contemplated to be formed by a press fit relationship between the rounded end 128 of tab 126 and the inside surfaces of curved projection 130.

In Figure 22, there is illustrated a further embodiment of a hinge which is generally similar to that shown in Figure 14. The hinge 134 is formed between a first member 136 and a second member 138. It is contemplated that the first and second member include engaging projections which are formed around a central pin 140. The upward rotation of second member 138 about the hinge 134 is shown in phantom.

In Figure 23, there is illustrated a further embodiment of a hinge connection. The hinge 142 is formed between a first planar member 144 and an L-shaped second member 146. The L-shaped second member 146 permits the upper surface of the second member 146 to engage the lower surface of the first planar member 144 when the hinge 142 is collapsed. The opening of hinge 142 is shown in phantom.

In Figure 24, there is illustrated a further hinge embodiment. The hinge 148 is formed between a first planar member 150 and a T-shaped second member 152. The hinge is formed on the central portion of the T-shape in a manner similar to that contemplated by the hinge 134 in Figure 22 and the hinge 142 in Figure 23. Again, the upper portion of the T-shaped member 152, when in inverted position, engages the lower surface of the planar member 150 to limit rotation about the hinge 148. The rotation of hinge 150 is illustrated in phantom.

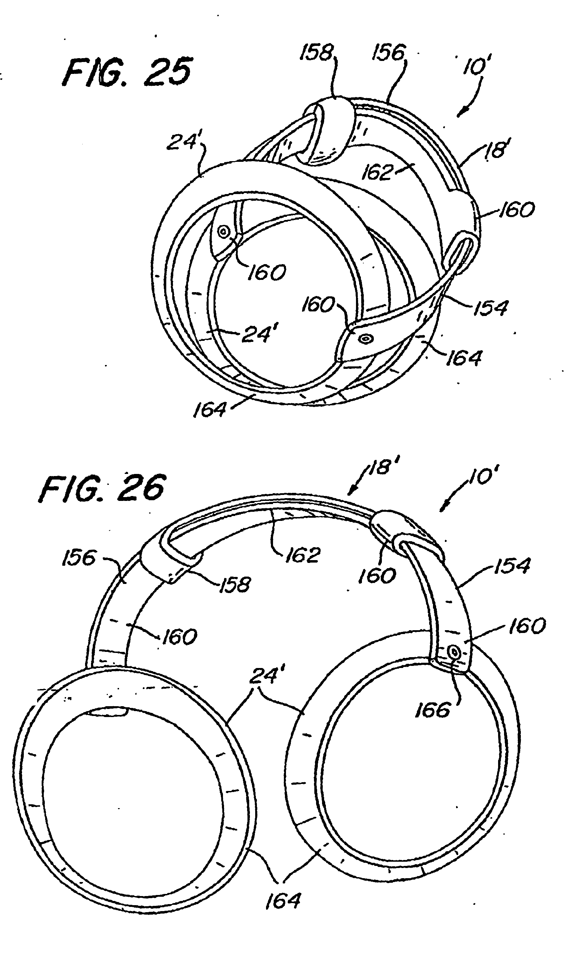

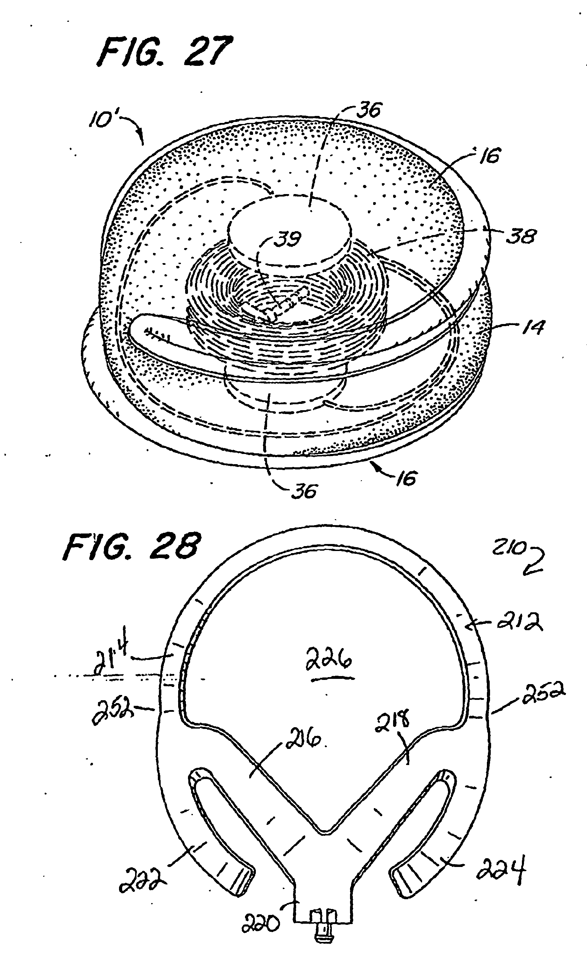

In Figures 25, 26 and 27, there is illustrated a collapsible ear protection device 10' which is shown in the open position (Figure 26) and in the closed position (Figures 25 and 27). For illustrative purposes, only the frame portion of the ear protection device 10' has been shown in Figures 25 and 26. However, a fabric covering similar to the embodiment shown in the figures discussed above is contemplated to be included and is shown in Figure 27.

The band 18' of device 10' generally includes a semi-circular first portion 154 and a semi-circular second portion 156 which engage one another to form a continuous arc. Engagement is formed by wrapping a portion of each member 156 and 154 around the other. The wrapping portion of the first semi-circular portion 154 is identified by the numeral 158. The wrapping portion of the second semi-circular member 156 is identified by the numeral 160. As an alternative to wrapping the two portions around one another, separate rings or bands may be wrapped around one end of the first member 154 (for example) and attached thereto. The rings will also be wrapped around the central portion of the second member 156. In final assembly, as illustrated in Figure 26, the end of the band 18', identified by the numeral 160, includes a single layer whereas the central portion 162 of the band 18' includes an overlapped double layer. The double layer portion may be shortened or elongated by sliding the two members 154 and 156 with respect to one another so as to move the wrapping portions 158 and 159 closer together. To shorten the overall length of the band 18', the two wrap portions 158 and 159 are moved further apart from one another (elongating the overlap 162 of the two portions, but shortening the overall length of the band 18').

In Figures 25 and 26, the frusto conical portion 24' forms the entirety of the ear cup 164. The connection between the ends 160 of the band 18' is preferably formed by an attachment head 166, similar to that specifically illustrated in Figure 16 (element 98; also illustrated in Figures 19-22), and a socket or opening in the ends 160 of the band 18'. As illustrated, the attachment bead is inserted in the opening in a press fit type relationship. Alternatively, the ends 160 could include an inverted V-shaped notch with a rounded opening at the top. The shaft of the attachment head in this contemplated embodiment would be slid into the notch and retained within the rounded opening, again in a press fit type relationship. Another possibility is a "figure 8" type opening with one side thereof being larger than the other. In this form, the enlarged diameter of the head portion can be inserted into the larger opening and then the shaft portion slid into the smaller portion of the opening (again in a press fit relationship). Other forms of detachable fastening and non-detachable fastening (such as a rivet) are contemplated. The intent of this type attachment is to permit the frusto conical portion 24' to rotate about the attachment head 166 and the end of the band 18'.

As is more particularly illustrated in Figure 27, one frusto conical portion 24' formed within the car covering portion 16' may be rotated so as to flatten against the opposite ear covering portion 16' (which is also rotated) with the central portion 14 (which covers band 18') forming a circular (or at least semi-circular) side wall between the parallel car covering portions. In this manner, the collapsed ear covering device 10' can retain the speaker wire 38 and male jack 39 (each shown in phantom). The speakers 36 (also shown in phantom) are retained within the ear covering device within the pocket or cavity formed by the central opening 62 within the central fabric portion (Figure 8E) and the respective outer shell 46 (Figure 8C) and inner ear members 64 (Figure 8F).

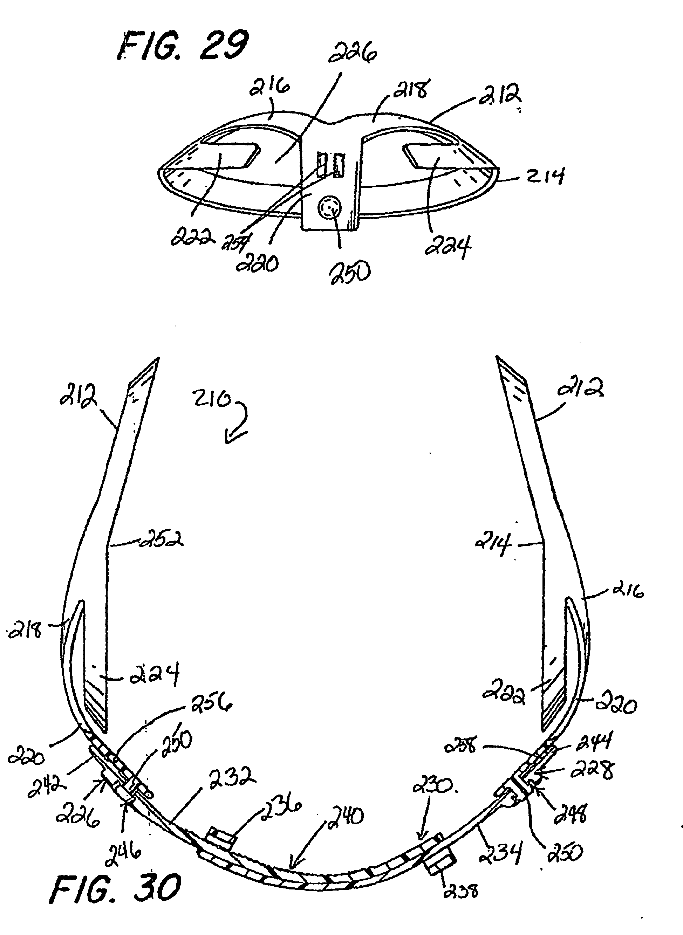

In Figures 28-30, there is shown an alternate embodiment of a frame for an ear protection device. The frame of this embodiment is generally labeled with the numeral 210. In Figures 28 and 29, there is particularly illustrated an ear cup frame member 212. The ear cup frame 212 includes a modified ear cavity having a semi-circular frame 214 at one end, two support flanges 216, 218 formed as a central V-shaped portion, an attachment portion or flange 220, and two cup extension portions 222 and 224. The modified ear cavity is generally shown in Figure 28. The semi-circular portion 214 defines, along with the support flanges 216, 218, a central opening 226. The attachment flange 220 extends outwardly from the vertex of the V-shape of the support flanges 216, 218.

As illustrated more particularly in Figures 29 and 30, the outline of the cavity of ear cup 212 is generally formed by the cup extensions 222, 224, along with the semi-circular frame portion 212. The support flanges 216, 218 project above the ear cop to further define the cavity for receiving an ear. Thus, the frame member 212 forms a open area defined by the height of the semi-circular portion 214 and the cup extensions 222 and 224, as well as the projection of the support flanges 216, 218 thereabove.

As shown in Figure 30, the frame 210 includes a band 230 which is comprised of a first curved portion 232 and a second curved portion 234. The two band portions 232, 234 overlap and engage one another by means of passageways 236, 238. Passageway 236 is positioned at one end of band portion 234, which is opposite band end 228. Passageway 238, formed as part of the band portion 232, is positioned at the opposite end of band 234 from attachment end 226. Band portion 232 on its inside surface includes a series of raised bumps or ratchet teeth 240 which form a resistance to the sliding movement of passageway 236 of band portion 234 when the expansion of the overall length of the band 230 is desired. The passageways 236 and 238 include a lower portion in which the opposing band slides. As can be seen, the thickness of the band ends 226, 228 increases in the area of the openings 246, 248. An upper portion is included in the passageways which permits the passage of the passageway 236 over the end 226 of band portion 232 and, similarly, the passage of passageway 238 over the band end 228 of band portion 234. Each band end 226 and 228 includes a projecting tab 242 and 244, respectively, which extends beyond the openings 246 and 248, respectively, which receive the attachment head 250 of the two ear cup frame members 212.

The connection between the attachment head 250 and the openings 246 and 248 in the band portions 232 and 234, respectively, permits the rotation of the ear cup around the axis of the attachment head 250. When the band ends 226, 228 are aligned with the attachment flange 220, the tabs 242, 244 form a moment arm which translates the spring force, created by the curvature of the band 230, to the ear cup frame members. This permits the ear cup frame member to flex and engage around the ear of the wearer. The semi-circular portion 214 flexes with respect to the more rigid structure of the support flanges 216, 218 due to the "twist" line created by the relief area 252 on opposite sides of the ear cup frame 212. In addition, the attachment flange 220 is provided with raised bumps 254 on the outside surface thereof. The raised bumps define a slot therebetween in which is engaged a bump 256 on the inside surface of band end 242 of band portion 232 or a bump 258 on end 228 of band portion 234. The engagement of bumps 256, 258 within the slot formed by the raised portions 254 on attachment flange 220 secures the ear cup frame member 212 in alignment with band 230. This engagement can be overcome by a slight rotation of the car cup 212 with respect to the band 230.

The overall structure of the embodiment shown in Figures 28-30 is contemplated to be generally larger than those shown in the earlier embodiments. In addition, the cavity of the ear cup frame member 212 is contemplated to have a more semi-circular interior due to the inclusion of the support flanges 216 and 218 and due to their relationship with the attachment flange 220.

The frame embodiment 210 shown in Figures 28-30 is contemplated to include a fabric covering similar to those discussed above. In addition, speakers, such as those shown in Figures 6, 7b and 27, may be included within the interior of the ear covering device 210.

The frame of this or any embodiment may be made of a Crastin® material which is manufactured by the DuPont Company. The Crastin® material is considered less suspectable to changes in moisture than a typical nylon material and is not severely effected by changes in temperature. It is contemplated that the characteristics of this material and its higher flex modulus will enable the frame to be relatively thinner than would be possible by a the use of nylon material, while still accomplishing the desired characteristics of the present invention.

The present invention may be embodied in other specific forms without departing from the spirit or essential attributes thereof and, accordingly, reference should be made to the appended claims, rather than to the foregoing specification, as indicating the scope of the invention.

## Claims

1. A covering device (10) to be worn over the ears of an individual (12) and extending around the band of the individual's head or neck, comprising:
a band portion (14, 18, 230) having a central curved portion (32, 162) and two opposite end portions (26, 160);
a first ear cup (20, 164) and a second ear cup (20, 164), each ear cup (20, 164) being coupled to an end portion of the band portion (14, 18, 230),
each ear cup (20, 164) having a central opening (34) therein, the curvature of the band portion (14, 18, 230) and the ear cups (20, 164) directing the ear cups (20, 164) inwardly toward each other from the opposite end portions (26, 160) of the band portion (14, 18, 230); and
fabric (40) covering the band portion (14, 18, 230) and the ear cups (20, 164), **characterized in that** each ear cup (20, 164) includes a cup portion defining at least a portion of the opening (34), and each cup portion has a frusto-conical shape.

2. The covering device (10) as claimed in claim 1, wherein each ear cup (20, 164) includes an attachment portion that is coupled to the band portion (14, 18, 230).

3. The covering device (10) as claimed in any one of claims 1 or 2, wherein each ear cup (20, 164) includes a cup portion defining at least a portion of the opening (34), and each attachment portion is integrally formed with each cup portion at an oblique angle thereto.

4. The covering device (10) as claimed in any one of claims 1 to 3, wherein the band portion (18, 230) includes a first curved portion and a second curved portion, one end of the first curved portion overlapping and slidably attached to one end of the second curved portion such that the relative overall length of the band portion (18, 230) may be adjusted.

5. The covering device (10) as claimed in any one of claims 1 to 4, wherein the ear cups (20, 164) are pivotally coupled to the band portion, each of the ear cups (20, 164) being pivotable with respect to the band portion (14, 18, 230) between a collapsed position and an expanded position.

6. The covering device (10) as claimed in claim 5, further comprising:
a positioner having a first portion and a second portion, the first portion of the positioner being disposed on the band portion, the second portion of the positioner being disposed on the first ear cup (20, 164), the first portion of the positioner being configured to cooperate with the second portion of the positioner when the first ear cup is disposed in its expanded position.

7. The covering device (10) as claimed in claim 6, wherein the second portion of the positioner includes a first protrusion and a second protrusion spaced from the first protrusion, the first portion of the positioner is configured to be disposed between the first protrusion and the second protrusion when the first ear cup (20, 164) is disposed in its expanded position.

8. The covering device (10) as claimed in claim 7, wherein the second portion of the positioner is disposed on the attachment portion of the first ear cup (20, 164).

9. The covering device (10) as claimed in any one of claims 1 to 8, further comprising:
an elongate member extending through at least a portion of the first ear cup and at least a portion of the band portion (14, 18, 230) to pivotally couple the first ear cup (20, 164) to the band portion (14, 18, 230).

10. The covering device (10) as claimed in any one of claims 1 to 9,
wherein the fabric (40) forms a cavity, and the band portion (14, 18, 230), the first ear cup (20, 164) and the second ear cup (20, 164) are disposed within the cavity formed by the fabric (40).

11. The covering device (10) as claimed in any one of claims 1 to 9,
wherein the fabric (40) includes a membrane that covers a side of the band portion (14, 18, 230) and a side of each of the ear cups (20, 164).

12. The covering device (10) as claimed in any one of claims 1 to 9,
wherein the fabric (40) includes fabric covering the band portion and fabric covering the first ear cup (20, 164), and the fabric covering the band portion (14, 18, 230) is coupled to the fabric covering the first ear cup (20, 164).

13. The covering device (10) as claimed in any one of claims 1 to 9,
wherein the fabric (40) includes a first fabric portion and a second fabric portion, and the first fabric portion and the second fabric portion are sewn together with a single seam.

14. The covering device (10) as claimed in any one of claims 1 to 9,
wherein the band portion (14, 18, 230) has an inner side and an outer side, each ear cup (20, 164) has an inner side and an outer side, and the fabric (40) covers both the inner side and the outer side of the band portion (14, 18, 230) and covers both the inner side and the outer side of each ear cup (20, 164).

15. The covering device (10) according to claim 1, wherein the fabric (40) comprises:
a plurality of fabric members including a first fabric member (58), a second fabric member (64), a third fabric member (64), and a fourth fabric member 52) collectively defining a cavity; the band portion (14, 18, 230), the first ear cup (20, 164), and the second ear cup (20, 164) being disposed within the cavity, the first fabric member (58) being attached to the second fabric member (64) and the third fabric member (64).

16. The covering device (10) as claimed in claim 15, wherein the second fabric member overlaps at least a portion of the third fabric member and at least a portion of the first fabric member.

17. The covering device (10) as claimed in any one of claims 15 or 16, wherein the fourth fabric member overlaps at least a portion of the third fabric member and at least a portion of the first fabric member.

18. The covering device (10) as claimed in any one of claims 15 to 17,
wherein the first ear cup (20, 164) includes a first end portion and a second end portion opposite its first end portion, the first end portion of the first ear cup (20, 164) is coupled to the band portion (14, 18, 230), the second ear cup (20, 164) includes a first end portion and a second end portion opposite its first end portion, the first end portion of the second ear cup (20, 164) is coupled to the band portion (14, 18, 230), the first fabric member has a length no less than a distance between the second end portion of the first ear cup (20, 164) to the second end portion of the second ear cup (20, 164).

19. The covering device (10) as claimed in any one of claims 15 to 18,
wherein at least a portion of a perimeter of the first fabric member is attached to at least a portion of a perimeter of the second fabric member, at least a portion of a perimeter of a third fabric member is attached to at least a portion of a perimeter of the first fabric member.

20. The covering device (10) as claimed in any one of claims 15 to 19,
wherein the band portion (14, 18, 230) has a first side and a second side opposite the first side of the band portion (14, 18, 230), the second fabric member is disposed such that the first side of the band portion (14, 18) is disposed between the second fabric member and the second side of the band portion (14, 18, 230), the third fabric member is disposed such that the first side of the band portion (14, 18, 230) is disposed between the third fabric member and the second side of the band portion (14, 18, 230), the second fabric member overlapping the third fabric member.

21. The covering device (10) as claimed in claim 20, wherein the first fabric member is disposed such that the second side of the band portion (14, 18, 230) is disposed between the first fabric member and the first side of the band portion (14, 18, 230), the fourth fabric member is disposed such that the first side of the band portion (14, 18, 230) is disposed between the fourth fabric ember and the second side of the band portion (14, 18, 230), the collective outer perimeter of the second fabric member, the third fabric member, and the fourth fabric member substantially corresponds to the outer perimeter of the first fabric member.

22. The covering device (10) according to claim 1, wherein:
the band portion (18, 230) has a first curved portion (154, 232) and a second curved portion (156, 234), one end of each of the first and second curved portions overlapping and slidably attached to one another such that the relative overall length of the band may be adjusted; and
one of the ear cups (20, 164) being attached to the end of the first curved portion (154, 232) opposite its overlapping end and the other of the ear cups (20, 164) being attached to the end of the second curved portion (156, 234) opposite its overlapping end , the ear cups (20, 164) including a cup portion having the opening (34) therein, the attachment of the ear cups (20, 164) to the band permitting rotation of the cup portions about the attachment.

23. The covering device (10) as claimed in claim 22, wherein the band portion (18, 230) includes a first curved portion (154, 232) and a second curved portion (156, 234), one end of each of the first and second curved portions overlapping and slidably attached to one another such that the relative overall length of the band portion (18, 230) may be adjusted.

24. The covering device (10) as claimed in any one of claims 22 or 23, wherein each ear cup includes two support flanges and a semi-circular frame portion that is coupled to and extends between opposite projections of the support flanges, the semi-circular portion and the support flanges defining the opening (34).

25. The covering device (10) as claimed in any one of claims 22 to 24, wherein each ear cup (20, 164) includes attachment flange projecting from the support flanges in a direction opposite the semi-circular portion, and the attachment flange of each ear cup (20, 164) being coupled to the band portion (18, 230).

26. The covering device (10) as claimed in any one of claims 22 to 25, wherein the two ear cup portions rotatably engage into a position at the free ends of the first and second curved portions so that the ear cup portions are aligned with the band portion (18, 230).

27. The covering device (10) as claimed in any one of claims 22 to 25,
wherein the band portion (18, 230) and the support flanges of each ear cup direct the corresponding ear cup inwardly toward the other ear cup to maintain position of the covering device on the individual's head.

28. The covering device (10) as claimed in any one of claims 22 to 27,
wherein the fabric (40) includes a plurality of fabric pieces.

29. The covering device (10) as claimed in any one of claims 22 to 28,
wherein the band portion (18, 230) and the ear cups (20, 164) each include an inner side and an outer side, and the fabric (40) covers both the inner side and the outer side of the band portion (18, 230) and the ear cups (20, 164).

## Patentansprüche

1. Bedeckungsvorrichtung (10), die über den Ohren eines Individuums (12) getragen werden soll, und die sich um ein Band des Kopfs oder des Nackens des Individuums erstreckt, die folgendes umfasst:
einen Bandbereich (14, 18, 230) mit einem zentralen gekrümmten Bereich (32, 162) und mit zwei gegenüberliegenden Endbereichen (26, 160);
eine erste Ohrschale (20, 164) und eine zweite Ohrschale (20, 164), wobei jede Ohrschale (20, 164) mit einem Endbereich des Bandbereiches (14, 18, 230) verbunden ist,
wobei jede Ohrschale (20, 164) eine zentrale Öffnung (34) darin aufweist, wobei die Krümmung des Bandbereiches (14, 18, 230) und der Ohrschalen (20, 164) die Ohrschalen (20, 164) zueinander nach innen von gegenüberliegenden Endbereichen (26, 160) des Bandbereiches (14, 18, 230) richten; und
wobei ein Gewebe (40) den Bandbereich (14, 18, 230) und die Ohrschalen (20, 164) bedeckt,
**dadurch gekennzeichnet, dass**
jede Ohrschale (20, 164) einen Schalenbereich einschließt, der zumindest einen Teil der Öffnung (34) definiert, und dass jeder Schalenbereich eine kegelstumpförmige Gestalt aufweist.

2. Bedeckungsvorrichtung (10), wie sie in Anspruch 1 beansprucht wird, wobei jede Ohrschale (20, 164) einen Anfügebereich einschließt, der an den Bandbereich (14, 18, 230) gekoppelt ist.

3. Bedeckungsvorrichtung (10), wie sie in irgendeinem einem der Ansprüche 1 oder 2 beansprucht wird, wobei jede Ohrschale (20, 164) einen Schalenbereich einschließt, der mindestens einen Bereich der Öffnung (34) definiert, und wobei jeder Anfügebereich integral mit jedem Schalenbereich unter einem schiefen Winkel dazu ausgebildet ist.

4. Bedeckungsvorrichtung (10) wie sie in irgend einem der Ansprüche 1 bis 3 beansprucht wird, wobei der Bandbereich (18, 230) einen ersten gekrümmten Bereich und einen zweiten gekrümmten Bereich einschließt, wobei ein Ende des ersten gekrümmten Bereiches überlappend und verschiebbar an einem Ende des zweiten gekrümmten Bereiches angebracht ist, so dass die relative Gesamtlänge des Bandbereiches (18, 230) eingestellt werden kann.

5. Bedeckungsvorrichtung (10), wie sie von irgendeinem der Ansprüche 1 bis 4 beansprucht wird, wobei die Ohrschalen (20, 164) drehbar an dem Bandbereich angekoppelt sind, wobei jede der Ohrschalen (20, 164) drehbar hinsichtlich des Bandbereiches (14, 18, 230) zwischen einer zusammengeklappten Position und einer auseinander geklappten Position ist.

6. Bedeckungsvorrichtung (10), wie sie in Anspruch 5 beansprucht wird, die weiterhin umfasst:
einen Positionierer mit einem ersten Bereich und einem zweiten Bereich, wobei der erste Bereich des Positionierers auf dem Bandbereich angeordnet ist, wobei der zweite Bereich des Positionierers auf der ersten Ohrschale (20, 164) angeordnet ist, wobei der erste Bereich des Positionierers konfiguriert ist, mit dem zweiten Bereich des Positionierers zusammen zu arbeiten, wenn die erste Ohrschale in ihrer auseinander geklappten Position angeordnet ist.

7. Bedeckungsvorrichtung (10), wie sie in Anspruch 6 beansprucht wird, wobei der zweite Bereich des Positionierers einen ersten Vorsprung und einen zweiten Vorsprung, der von dem ersten Vorsprung beabstandet ist, einschließt, wobei der erste Bereich des Positionierers konfiguriert ist, zwischen dem ersten Vorsprung und dem zweiten Vorsprung angeordnet zu werden, wenn die erste Ohrschale (20, 164) in ihrer auseinander geklappten Position angeordnet ist.

8. Bedeckungsvorrichtung (10), wie sie in Anspruch 7 beansprucht wird, wobei die zweite Position des Positionierers auf dem Anfügebereich der ersten Ohrschale (20, 164) angeordnet ist.

9. Bedeckungsvorrichtung (10), wie sie in irgend einem der Ansprüche 1 bis 8 beansprucht wird, die weiterhin umfasst:
ein längliches Element, das sich durch zumindest einen Bereich der ersten Ohrschale und zumindest einen Bereich des Bandbereiches (14, 18, 230) erstreckt, um die erste Oberschale (20, 164) an den Bandbereich (14, 18, 230) zu koppeln.

10. Bedeckungsvorrichtung (10), wie sie in irgend einem der Ansprüche 1 bis 9 beansprucht wird, wobei das Gewebe (40) eine Tasche bildet, und wobei der Bandbereich (14, 18, 230), die erste Ohrschale (20, 164) und die zweite Ohrschale (20, 164) in der Tasche, die durch das Gewebe (40) gebildet wird, angeordnet sind.

11. Bedeckungsvorrichtung (10), wie sie in irgend einem der Ansprüche 1 bis 9 beansprucht wird, wobei das Gewebe (40) eine Membran einschließt, die eine Seite des Bandbereiches (14, 18, 230) und eine Seite von jeder Ohrschale (20, 164) bedeckt.

12. Bedeckungsvorrichtung (10) wie sie in irgend einem der Ansprüche 1 bis 9 beansprucht wird, wobei das Gewebe (40) ein Gewebe, das den Bandbereich bedeckt, und ein Gewebe, das die erste Ohrschale (20, 164) bedeckt, einschließt, und das Gewebe, das den Bandbereich (14, 18, 230) bedeckt, ist an das Gewebe gekoppelt, das die erste Ohrschale (20, 164) bedeckt.

13. Bedeckungsvorrichtung nach Anspruch 10, wie sie in irgend einem der Ansprüche 1 bis 9 beansprucht wird, wobei das Gewebe (40) einen ersten Gewebebereich und einem zweiten Gewebebereich einschließt, und wobei der erste Gewebebereich und der zweite Gewebebereich mit einer einzelnen Naht zusammengenäht sind.

14. Bedeckungsvorrichtung (10), wie sie in irgend einem der Ansprüche 1 bis 9 beansprucht wird, wobei der Bandbereich (14, 18, 230) eine innere Seite und eine äußere Seite aufweist, wobei jede Ohrschale (20, 164) eine innere Seite und eine äußere Seite aufweist, und wobei das Gewebe (40) sowohl die innere Seite als auch die äußere Seite des Bandbereiches (14, 18, 230) bedeckt und sowohl die innere Seite als auch die äußere Seite jeder Ohrschale (20, 164) bedeckt.

15. Bedeckungsvorrichtung (10) nach Anspruch 1, wobei das Gewebe (40) umfasst:
eine Vielzahl von Gewebeelementen, einschließlich einem ersten Gewebeelement (50), einem zweiten Gewebeelement (64), einem dritten Gewebeelement (64), und einem vierten Gewebeelement (52), die gemeinsam eine Tasche definieren; wobei der Bandbereich (14, 18, 230), die erste Ohrschale (20, 164), und die zweite Ohrschale (20, 164) innerhalb der Tasche angeordnet sind, wobei das erste Gewebeelement (58) an dem zweiten Gewebeelement (64) und dem dritten Gewebeelement (64) angebracht ist.

16. Bedeckungsvorrichtung (10), wie sie in Anspruch 15 beansprucht wird, wobei das zweite Gewebeelement mindestens einen Teil des dritten Gewebeelementes und mindestens einen Teil des ersten Gewebeelementes überlappt.

17. Bedeckungsvorrichtung (10), wie sie von irgend einem der Ansprüche 15 oder 16 beansprucht wird, wobei das vierte Gewebeelement mindestens einen Teil des dritten Gewebeelementes und mindestens einen Teil des ersten Gewebeelementes überlappt.

18. Bedeckungsvorrichtung (10), wie sie von irgend einem der Ansprüche 15 bis 17 beansprucht wird, wobei die erste Ohrschale (20, 164) einen ersten Endbereich und einen zweiten Endbereich, der seinem ersten Endbereich gegenüber liegt, einschließt, wobei der erste Endbereich der ersten Ohrschale (20, 164) an den Bandbereich (14, 18, 230) gekoppelt ist,
wobei die zweite Ohrschale (20, 164) einen ersten Endbereich und einen zweiten Endbereich, der seinem ersten Endbereich gegenüber liegt, einschließt, wobei der erste Endbereich der zweiten Ohrschale (20, 164) an den Bandbereich (14, 18, 230) gekoppelt ist, wobei das erste Gewebeelement eine Länge aufweist, die nicht geringer ist als ein Abstand zwischen dem zweiten Endbereich der ersten Ohrschale (20, 164) und dem zweiten Endbereich der zweiten Ohrschale (20, 164).

19. Bedeckungsvorrichtung (10), wie sie in irgend einem der Ansprüche 15 bis 18 beansprucht wird, wobei mindestens ein Umfangsbereich des ersten Gewebeelementes an mindestens einem Umfangsbereich des zweiten Gewebeelementes angebracht ist, wobei mindestens ein Umfangsbereich eines dritten Gewebeelementes an mindestens einem Umfangsbereich des ersten Gewebeelementes angebracht ist.

20. Bedeckungsvorrichtung (10), wie sie von irgend einem der Ansprüche 15 bis 19 beansprucht wird, wobei der Bandbereich (14, 18, 230) eine erste Seite und eine zweite Seite, die der ersten Seite des Bandbereiches (14, 18, 230) gegenüberliegt, aufweist, wobei das zweite Gewebeelement so angeordnet ist, dass die erste Seite des Bandbereiches (14, 18) zwischen dem zweiten Gewebeelement und der zweiten Seite des Bandbereiches (14, 18, 230) angeordnet ist, wobei das dritte Gewebeelement so angeordnet ist, dass die erste Seite des Bandbereiches (14, 18, 230) zwischen dem dritten Gewebeelement und der zweiten Seite des Bandbereichs (14, 18, 230) angeordnet ist, wobei das zweite Gewebeelement das dritte Gewebeelement überlappt.

21. Bedeckungsvorrichtung (10), wie sie in Anspruch 20 beansprucht wird, wobei das erste Gewebeelement so angeordnet ist, dass die zweite Seite des Bandbereiches (14, 18, 230) zwischen dem ersten Gewebeelement und der ersten Seite des Bandbereiches (14, 18, 230) angeordnet ist, das vierte Gewebeelement so angeordnet ist, dass die erste Seite des Bandbereiches (14, 18, 230) zwischen dem vierten Gewebeelement und der zweiten Seite des Bandbereiches (14, 18, 230) angeordnet ist, wobei der gemeinsame äußere Umfang des zweiten Gewebeelementes, des dritten Gewebeelementes oder des vierten Gewebeelementes im Wesentlichen dem äußeren Umfang des ersten Gewebeelementes entspricht.

22. Bedeckungsvorrichtung (10) nach Anspruch 1, wobei:
der Bandbereich (18, 230) einen ersten gekrümmten Bereich (154, 232) und einen zweiten gekrümmten Bereich (156, 234) aufweist, wobei ein Ende jedes des ersten und zweiten gekrümmten Bereiches überlappend und verschiebbar aneinander angebracht sind, so dass die relative Gesamtlänge des Bandes eingestellt werden; und
eine der Ohrschalen (20, 164) an einem Ende des ersten gekrümmten Bereiches (154, 232) gegenüberliegend zu ihrem überlappenden Ende angebracht ist und die andere der Ohrschalen (20, 164) an dem Ende des zweiten gekrümmten Bereiches (156, 234) gegenüber seinem überlappenden Ende angebracht ist, wobei die Ohrschalen (20, 164) einen Schalenbereich mit der Öffnung (34) darin einschließen, wobei das Anbringen der Ohrschalen (20, 164) an dem Band eine Drehung der Schalenbereiche um die Befestigung erlauben.

23. Bedeckungsvorrichtung (10), wie sie in Anspruch 22 beansprucht wird, wobei der Bandbereich (18, 230) einen ersten gekrümmten Bereich (154, 232) und einen zweiten gekrümmten Bereich (156, 234) einschließt, wobei ein Ende von jedem der ersten und zweiten gekrümmten Bereiche überlappend und verschiebbar aneinander angebracht sind, so dass die relative Gesamtlänge des Bandbereiches (18, 230) eingestellt werden kann.

24. Bedeckungsvorrichtung (10), wie sie von irgend einem der Ansprüche 22 oder 23 beansprucht wird, wobei jede Ohrschale zwei Stützflansche und einen halbkreisförmigen Rahmenbereich, der sich zwischen gegenüberliegenden Vorsprüngen der Stützflansche erstreckt und daran angekoppelt ist, einschließt, wobei der halbkreisförmige Bereich und die Stützflansche die Öffnung (34) definieren.

25. Bedeckungsvorrichtung (10), wie sie in irgend einem der Ansprüche 22 bis 24 beansprucht wird, wobei jede Ohrschale (20, 164) Befestigungsflansche einschließt, die von den Stützflanschen in einer Richtung gegenüberliegend dem halbkreisförmigen Bereich vorstehen, und wobei der Befestigungsflansch jeder Ohrschale (20, 164) an den Bandbereich (18, 230) gekoppelt ist.

26. Bedeckungsvorrichtung (10), wie sie in irgend einem der Ansprüche 22 bis 25 beansprucht wird, wobei die zwei Ohrschalenbereiche rotierbar in einer Position an den freien Enden der ersten und zweiten gekrümmten Bereiche einrasten, so dass die Ohrschalenbereiche zu dem Bandbereich (18, 230) ausgerichtet sind.

27. Bedeckungsvorrichtung (10), wie sie in irgend einem der Ansprüche 22 bis 25 beansprucht wird, wobei der Bandbereich (18, 230) und die Stützflansche von jeder Ohrschale die entsprechende Ohrschale nach innen richten zu der anderen Ohrschale, um die Position der Bedeckungsvorrichtung auf dem Kopf des Individuums zu halten.

28. Bedeckungsvorrichtung (10), wie sie in irgend einem der Ansprüche 22 bis 27 beansprucht wird, wobei das Gewebe (40) eine Vielzahl von Gewebestücken einschließt.

29. Bedeckungsvorrichtung (10), wie sie in irgend einem der Ansprüche 22 bis 28 beansprucht wird, wobei der Bandbereich (18, 230) und die Ohrschalen (20, 164) jeweils eine innere Seite und eine äußere Seite einschließen, und wobei das Gewebe (40) sowohl die innere Seite als auch die äußere Seite des Bandbereiches (18, 230) und die Ohrschalen (20, 164) bedeckt.

## Revendications

1. Dispositif de couverture (10) à porter par un individu (12) sur ses oreilles et s'étendant autour de la bande de la tête ou du cou de l'individu, comprenant :
une partie de bande (14, 18, 230) ayant une partie incurvée centrale (32, 162) et deux parties d'extrémité opposées (26, 160) ;
une première oreillette (20, 164) et une seconde oreillette (20, 164), chaque oreillette (20, 164) étant couplée à une partie d'extrémité de la partie de bande (14, 18, 230),
chaque oreillette (20, 164) renfermant une ouverture centrale (34), la courbure de la partie de bande (14, 18, 230) et les oreillettes (20, 164) dirigeant les oreillettes (20, 164) vers l'intérieur les unes vers les autres depuis les parties d'extrémité opposées (26, 160) de la partie de bande (14, 18, 230) ; et
un tissu (40) couvrant la partie de bande (14, 18, 230) et les oreillettes (20, 164) **caractérisé en ce que**
chaque oreillette (20, 164) inclut une partie d'oreillette définissant au moins une partie de l'ouverture (34), et chaque partie d'oreillette ayant une forme tronconique.

2. Dispositif de couverture (10) selon la revendication 1, dans lequel chaque oreillette (20, 164) inclut une partie d'attache qui est couplée à la partie de bande (14, 18, 230).

3. Dispositif de couverture (10) selon l'une quelconque des revendications 1 ou 2, dans lequel chaque oreillette (20, 164) inclut une partie d'oreillette définissant au moins une partie de l'ouverture (34), et chaque partie d'attache est formée solidairement avec chaque partie d'oreillette selon un angle oblique par rapport à celle-ci.

4. Dispositif de couverture (10) selon l'une quelconque des revendications 1 à 3, dans lequel la partie de bande (18, 230) inclut une première partie incurvée et une seconde partie incurvée, une extrémité de la première partie incurvée chevauchant et étant attachée de façon coulissante à une extrémité de la seconde partie incurvée de telle sorte que la longueur globale relative de la partie de bande (18, 230) peut être ajustée.

5. Dispositif de couverture (10) selon l'une quelconque des revendications 1 à 4, dans lequel les oreillettes (20, 164) sont couplées en pivotement à la partie de bande, chacune des oreillettes (20, 164) étant pivotante par rapport à la partie de bande (14, 18, 230) entre une position affaissée et une position déployée.

6. Dispositif de couverture (10) selon la revendication 5, comprenant en outré :
un dispositif de positionnement comportant une première partie et une seconde partie, la première partie du dispositif de positionnement étant disposée sur la partie de bande, la seconde partie du dispositif de positionnement étant disposée sur la première oreillette (20, 164), la première partie du dispositif de positionnement étant configurée pour coopérer avec la seconde partie du dispositif de positionnement lorsque la première oreillette est disposée dans sa position déployée.

7. Dispositif de couverture (10) selon la revendication 6, dans lequel la seconde partie du dispositif de positionnement inclut une première protubérance et une seconde protubérance espacée de la première protubérance, la première partie du dispositif de positionnement est configurée pour être disposée entre la première protubérance et la seconde protubérance lorsque la première oreillette (20, 164) est disposée dans sa position déployée.

8. Dispositif de couverture (10) selon la revendication 7, dans lequel la seconde partie du dispositif de positionnement est disposée sur la partie d'attache de la première oreillette (20, 164).

9. Dispositif de couverture (10) selon l'une quelconque des revendications 1 à 8, comprenant en outré :
un organe allongé s'étendant à travers au moins une partie de la première oreillette et au moins une partie de la partie de bande (14, 18, 230) pour coupler en pivotement la première oreillette (20, 164) à la partie de bande (14, 18, 230).

10. Dispositif de couverture (10) selon l'une quelconque des revendications 1 à 9, dans lequel le tissu (40) forme une cavité, et la partie de bande (14, 18, 230), la première oreillette (20, 164) et la seconde oreillette (20, 164) sont disposées dans la cavité formée par le tissu (40).

11. Dispositif de couverture (10) selon l'une quelconque des revendications 1 à 9, dans lequel le tissu (40) inclut une membrane qui couvre un côté de la partie de bande (14, 18, 230) et un côté de chacune des oreillettes (20, 164).

12. Dispositif de couverture (10) selon l'une quelconque des revendications 1 à 9, dans lequel le tissu (40) inclut un tissu couvrant la partie de bande et le tissu couvrant la première oreillette (20, 164), et le tissu couvrant la partie de bande (14, 18, 230) est couplé au tissu couvrant la première oreillette (20, 164).

13. Dispositif de couverture (10) selon l'une quelconque des revendications 1 à 9, dans lequel le tissu (40) inclut une première partie de tissu et une seconde partie de tissu, et la première partie de tissu et la seconde partie de tissu sont cousues ensemble avec une seule couture.

14. Dispositif de couverture (10) selon l'une quelconque des revendications 1 à 9, dans lequel la partie de bande (14, 18, 230) comporte un côté interne et un côté externe, chaque oreillette (20, 164) a un côté interne et un côté externe, et le tissu (40) couvre à la fois le côté interne et le côté externe de la partie de bande (14, 18, 230) et couvre à la fois le côté interne et le côté externe de chaque oreillette (20, 164).

15. Dispositif de couverture (10) selon la revendication 1, dans lequel le tissu (40) comprend :
une pluralité d'organes de tissu incluant un premier organe de tissu (58), un deuxième organe de tissu (64), un troisième organe de tissu (64) et un quatrième organe de tissu (52) définissant collectivement une cavité ; la partie de bande (14, 18, 230), la première oreillette (20, 164), et la seconde oreillette (20, 164) étant disposées dans la cavité, le premier organe de tissu (58) étant attaché au deuxième organe de tissu (64) et au troisième organe de tissu (64).

16. Dispositif de couverture (10) selon la revendication 15, dans lequel le deuxième organe de tissu chevauche au moins une partie du troisième organe de tissu et au moins une partie du premier organe de tissu.

17. Dispositif de couverture (10) selon l'une quelconque des revendications 15 ou 16, dans lequel le quatrième organe de tissu chevauche au moins une partie du troisième organe de tissu et au moins une partie du premier organe de tissu.

18. Dispositif de couverture (10) selon l'une quelconque des revendications 15 à 17, dans lequel la première oreillette (20, 164) inclut une première partie d'extrémité et une seconde partie d'extrémité opposée à sa première partie d'extrémité, la première partie d'extrémité de la première oreillette (20, 164) étant couplée à la partie de bande (14, 18, 230), la seconde oreillette (20, 164) inclut une première partie d'extrémité et une seconde partie d'extrémité opposée à sa première partie d'extrémité, la première partie d'extrémité de la seconde oreillette (20, 164) est couplée à la partie de bande (14, 18, 230), le premier organe de tissu a une longueur non inférieure à une distance entre la seconde partie d'extrémité de la première oreillette (20, 164) à la seconde partie d'extrémité de la seconde oreillette (20, 164).

19. Dispositif de couverture (10) selon l'une quelconque des revendications 15 à 18, dans lequel au moins une partie d'un périmètre du premier organe de tissu est attachée à au moins une partie d'un périmètre du deuxième organe de tissu, au moins une partie d'un périmètre d'un troisième organe de tissu est attachée à au moins une partie d'un périmètre du premier organe de tissu.

20. Dispositif de couverture (10) selon l'une quelconque des revendications 15 à 19, dans lequel la partie de bande (14, 18, 230) comporte un premier côté et un second côté opposé au premier côté de la partie de bande (14, 18, 230), le deuxième organe de tissu est disposé de telle sorte que le premier côté de la partie de bande (14, 18) est disposé entre le deuxième organe de tissu et le second côté de la partie de bande (14, 18, 230), le troisième organe de tissu est disposé de telle sorte que le premier côté de la partie de bande (14, 18, 230) est disposé entre le troisième organe de tissu et le second côté de la partie de bande (14, 18, 230), le deuxième organe de tissu chevauchant le troisième organe de tissu.

21. Dispositif de couverture (10) selon la revendication 20, dans lequel le premier organe de tissu est disposé de telle sorte que le second côté de la partie de bande (14, 18, 230) est disposé entre le premier organe de tissu et le premier côté de la partie de bande (14, 18, 230), le quatrième organe de tissu est disposé de telle sorte que le premier côté de la partie de bande (14, 18, 230) est disposé entre le quatrième organe de tissu et le second côté de la partie de bande (14, 18, 230), le périmètre externe collectif du deuxième organe de tissu, du troisième organe de tissu et du quatrième organe de tissu correspond sensiblement au périmètre externe du premier organe de tissu.

22. Dispositif de couverture (10) selon la revendication 1, dans lequel
la partie de bande (18, 230) comporte une première partie incurvée (154, 232) et une seconde partie incurvée (156, 234), une extrémité de chacune des première et seconde parties incurvées chevauchant et étant attachée de façon coulissante à une autre extrémité de telle sorte que la longueur globale relative de la bande peut être ajustée ; et
une des oreillettes (20, 164) étant attachée à l'extrémité de la première partie incurvée (154, 232) opposée à son extrémité chevauchante et l'autre des oreillettes (20, 164) étant attachée à l'extrémité de la seconde partie incurvée (156, 234) opposée à son extrémité chevauchante, les oreillettes (20, 164) incluant une partie d'oreillette renfermant l'ouverture (34), l'attache des oreillettes (20, 164) à la bande permettant une rotation des parties d'oreillettes autour de l'attache.

23. Dispositif de couverture (10) selon la revendication 22, dans lequel la partie de bande (18, 230) inclut une première partie incurvée (154, 232) et une seconde partie incurvée (156, 234), une extrémité de chacune des première et seconde parties incurvées chevauchant et étant attachée de façon coulissante à une autre extrémité de telle sorte que la longueur globale relative de la partie de bande (18, 230) peut être ajustée.

24. Dispositif de couverture (10) selon l'une quelconque des revendications 22 ou 23, dans lequel chaque oreillette inclut deux brides de support et une partie de cadre semi-circulaire qui est couplée à, et s'étend entre, des saillies opposées des brides de support, la partie semi-circulaire et les brides de support définissant l'ouverture (34).

25. Dispositif de couverture (10) selon l'une quelconque des revendications 22 à 24, dans lequel chaque oreillette (20, 164) inclut une bride d'attache faisant saillie des brides de support dans une direction opposée à la partie semi-circulaire, et la bride d'attache de chaque oreillette (20, 164) étant couplée à la partie de bande (18, 230).

26. Dispositif de couverture (10) selon l'une quelconque des revendications 22 à 25, dans lequel les deux parties d'oreillettes s'accouplent en rotation dans une position au niveau des extrémités libres des première et seconde parties incurvées de telle sorte que les parties d'oreillettes sont alignées avec la partie de bande (18, 230).

27. Dispositif de couverture (10) selon l'une quelconque des revendications 22 à 25, dans lequel la partie de bande (18, 230) et les brides de support de chaque oreillette dirigent l'oreillette correspondante vers l'intérieur vers l'autre oreillette pour maintenir la position du dispositif de couverture sur la tête d'un individu.

28. Dispositif de couverture (10) selon l'une quelconque des revendications 22 à 27, dans lequel le tissu (40) inclut une pluralité de pièces de tissu.

29. Dispositif de couverture (10) selon l'une quelconque des revendications 22 à 28, dans lequel la partie de bande (18, 230) et les oreillettes (20, 164) incluent chacune un côté interne et un côté externe, et le tissu (40) couvre à la fois le côté interne et le côté externe de la partie de bande (18, 230) et les oreillettes (20, 164).
